# EUROPEAN PATENT APPLICATION

(11) **EP 3 476 346 A1**
(43) Date of publication of application: **01.05.2019**
(21) Application number: 18203186.4
(22) Date of filing: 29.10.2018
(51) Int. Cl.: A61B 18/14, A61B 18/12

(54) **SUCTION INSTRUMENT WITH BIPOLAR RF CUFF**

(30) Priority: 30.10.2017 US 201715797091
(71) Applicant: Acclarent, Inc., Irvine, CA 92618 (US)
(72) Inventor: PALUSHI, Jetmir, Irvine, CA California 92618 (US); FANG, Itzhak, Irvine, CA California 92618 (US); SALAZAR, Henry F., Irvine, CA California 92618 (US)
(74) Representative: Small, Gary James

(57) **Abstract**

A surgical instrument includes an elongate body configured to be inserted through the nasal cavity of a patient, and a plurality of electrodes spaced circumferentially about an exterior of the elongate body. The electrodes may be arranged at a distal end of the elongate body, and are configured to deliver bipolar RF energy to tissue for sealing the tissue. The elongate body may be in the form of a shaft having a lumen configured to draw fluid proximally through the surgical instrument with suction.

## Description

### BACKGROUND

Electrosurgical instruments utilize electrical energy for sealing tissue, and generally include a distally mounted end effector that can be configured for bipolar or monopolar operation. During bipolar operation, electrical current is provided through the tissue by active and return electrodes of the end effector. During monopolar operation, current is provided through the tissue by an active electrode of the end effector and a return electrode (*e.g*., a grounding pad) separately located on a patient's body. Heat generated by the current flowing through the tissue may form hemostatic seals within the tissue and/or between tissues, and thus may be particularly useful for sealing blood vessels, for example. The end effector of some electrosurgical devices may also include a cutting member that is movable relative to the tissue and the electrodes to transect the tissue.

Electrical energy applied by an electrosurgical device can be transmitted to the instrument by a generator coupled with the instrument. The electrical energy may be in the form of radio frequency ("RF") energy, which is generally in the frequency range of approximately 300 kilohertz (kHz) to 1 megahertz (MHz). In use, an electrosurgical device can transmit lower frequency RF energy through tissue, which causes ionic agitation, or friction, in effect resistive heating, thereby increasing the temperature of the tissue. Because a sharp boundary is created between the affected tissue and the surrounding tissue, surgeons can operate with a high level of precision and control, without sacrificing un-targeted adjacent tissue. The low operating temperatures of RF energy is useful for removing, shrinking, or sculpting soft tissue while simultaneously sealing blood vessels. RF energy works particularly well on connective tissue, which is primarily comprised of collagen and shrinks when contacted by heat.

An example of an RF electrosurgical device is the ENSEAL® Tissue Sealing Device by Ethicon Endo-Surgery, Inc., of Cincinnati, Ohio. Further examples of electrosurgical devices and related concepts are disclosed in U.S. Pat. No. 6,500,176 entitled "Electrosurgical Systems and Techniques for Sealing Tissue," issued Dec. 31, 2002, the disclosure of which is incorporated by reference herein; U.S. Pat. No. 7,112,201 entitled "Electrosurgical Instrument and Method of Use," issued Sep. 26, 2006, the disclosure of which is incorporated by reference herein; U.S. Pat. No. 7,125,409, entitled "Electrosurgical Working End for Controlled Energy Delivery," issued Oct. 24, 2006, the disclosure of which is incorporated by reference herein; U.S. Pat. No. 7,169,146 entitled "Electrosurgical Probe and Method of Use," issued Jan. 30, 2007, the disclosure of which is incorporated by reference herein; U.S. Pat. No. 7,186,253, entitled "Electrosurgical Jaw Structure for Controlled Energy Delivery," issued Mar. 6, 2007, the disclosure of which is incorporated by reference herein; U.S. Pat. No. 7,189,233, entitled "Electrosurgical Instrument," issued Mar. 13, 2007, the disclosure of which is incorporated by reference herein; U.S. Pat. No. 7,220,951, entitled "Surgical Sealing Surfaces and Methods of Use," issued May 22, 2007, the disclosure of which is incorporated by reference herein; U.S. Pat. No. 7,309,849, entitled "Polymer Compositions Exhibiting a PTC Property and Methods of Fabrication," issued Dec. 18, 2007, the disclosure of which is incorporated by reference herein; U.S. Pat. No. 7,311,709, entitled "Electrosurgical Instrument and Method of Use," issued Dec. 25, 2007, the disclosure of which is incorporated by reference herein; U.S. Pat. No. 7,354,440, entitled "Electrosurgical Instrument and Method of Use," issued Apr. 8, 2008, the disclosure of which is incorporated by reference herein; U.S. Pat. No. 7,381,209, entitled "Electrosurgical Instrument," issued Jun. 3, 2008, the disclosure of which is incorporated by reference herein.

Additional examples of electrosurgical devices and related concepts are disclosed in U.S. Pat. No. 8,939,974, entitled "Surgical Instrument Comprising First and Second Drive Systems Actuatable by a Common Trigger Mechanism," issued Jan. 27, 2015, the disclosure of which is incorporated by reference herein; U.S. Pat. No. 9,161,803, entitled "Motor Driven Electrosurgical Device with Mechanical and Electrical Feedback," issued Oct. 20, 2015, the disclosure of which is incorporated by reference herein; U.S. Pub. No. 2012/0078243, entitled "Control Features for Articulating Surgical Device," published Mar. 29, 2012, the disclosure of which is incorporated by reference herein; U.S. Pat. No. 9,402,682, entitled "Articulation Joint Features for Articulating Surgical Device," issued Aug. 2, 2016, the disclosure of which is incorporated by reference herein; U.S. Pat. No. 9,089,327, entitled "Surgical Instrument with Multi-Phase Trigger Bias," issued July 28, 2015, the disclosure of which is incorporated by reference herein; U.S. Pat. No. 9,545,253, entitled "Surgical Instrument with Contained Dual Helix Actuator Assembly," issued Jan. 17, 2017, the disclosure of which is incorporated by reference herein; and U.S. Pat. No. 9,572,622, entitled "Bipolar Electrosurgical Features for Targeted Hemostasis," issued Feb. 21, 2017, the disclosure of which is incorporated by reference herein.

While various types of electrosurgical instruments have been made and used, it is believed that no one prior to the inventor(s) has made or used the invention described herein.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary surgical instrument having RF electrodes arranged at a distal end thereof, with a suction source and a power source shown schematically;
FIG. 2 depicts partial side sectional view of the surgical instrument of FIG. 1, showing fluid flow through a lumen of the instrument;
FIG. 3 depicts an enlarged perspective view of the distal end of the surgical instrument of FIG. 1;
FIG. 4A depicts a left sagittal sectional view of a portion of a patient's head, showing paranasal sinus structures; and
FIG. 4B depicts an enlarged left sagittal sectional view of the patient head of FIG. 4A, showing the distal end of the surgical instrument of FIG. 1 being inserted through an opening formed in the wall of the ethmoid bulla.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

For clarity of disclosure, the terms "proximal" and "distal" are defined herein relative to a surgeon, or other operator, grasping a surgical instrument having a distal end. The term "proximal" refers to the position of an element arranged closer to the surgeon, and the term "distal" refers to the position of an element arranged closer to the distal end of the surgical instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

As used herein, the terms "about" and "approximately" for any numerical values or ranges indicate a suitable dimensional tolerance that allows the part or collection of components to function for its intended purpose as described herein.

### I. Exemplary Surgical Instrument System

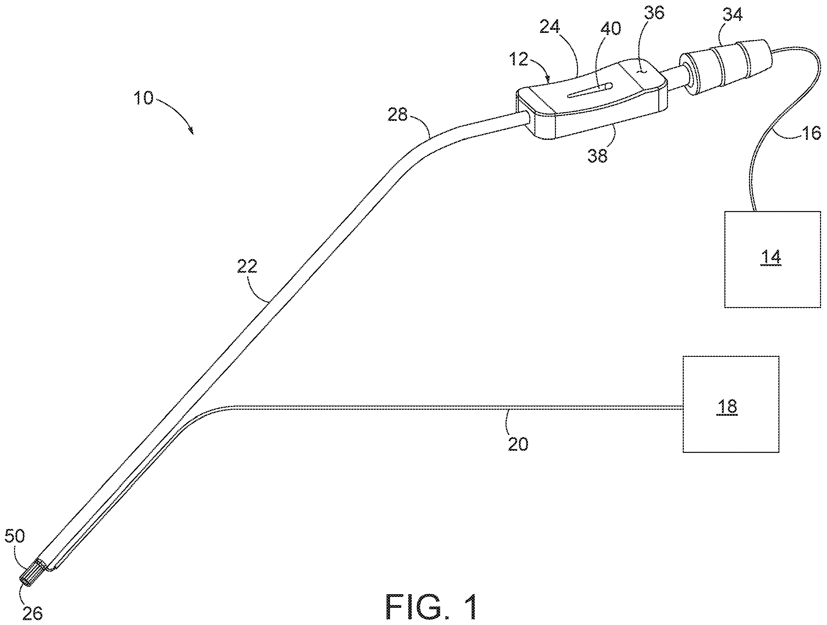

FIG. 1 shows an exemplary surgical instrument system (10) including a surgical instrument (12) configured to provide suction and bipolar RF energy for sealing tissue at a surgical site. As shown schematically, surgical system (10) further includes a suction source (14) fluidly coupled with surgical instrument (12) via a suction conduit (16), and a generator (18) electrically coupled with surgical instrument (12) via a wire (20). Suction source (14) may comprise a vacuum pump and a fluid reservoir, among other components known in the art, and is configured to provide suction sufficient to draw fluids (e.g., mucus, blood, etc.) and/or debris (e.g., tissue, bone fragments, etc.) from the surgical site proximally through surgical instrument (12). Generator (18) is configured to energize RF electrodes of surgical instrument (12) with bipolar RF energy for sealing tissue at the surgical site, as described in greater detail below.

### A. Exemplary Surgical Instrument Having RF Cuff

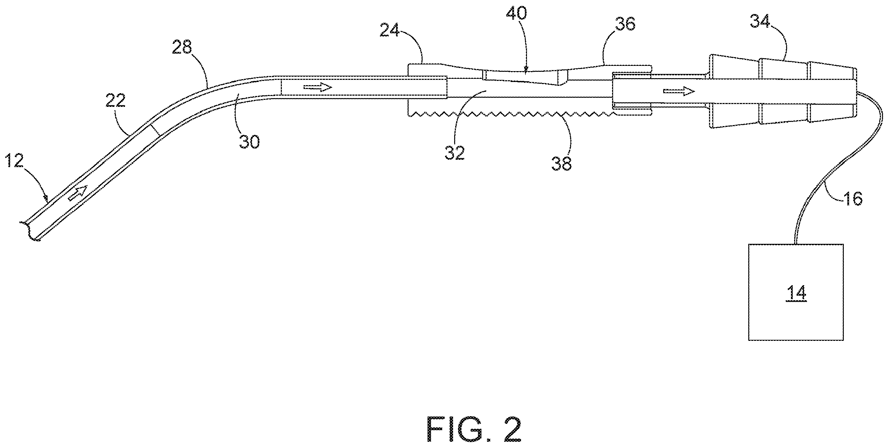

As shown in FIGS. 1 and 2, surgical instrument (12) of the present example comprises an elongate body in the form of a cannula shaft (22), and a grip portion (24) arranged at a proximal end portion of cannula shaft (22) such that cannula shaft (22) extends distally from grip portion (24). Cannula shaft (22) has an open distal end (26) and a bent region (28) formed just distally of grip portion (24). Bent region (28) defines a bend angle that is selected to facilitate insertion of distal end (26) in a patient by an operator grasping grip portion (24). Various suitable bend angles that may be provided will be apparent to those of ordinary skill in the art in view of the teachings herein. In the present example, cannula shaft (22) is formed with a rigid construction such that cannula shaft (22) maintains the bend angle of bent region (28) and does not buckle during insertion of cannula shaft (22) into a patient. By way of example only, cannula shaft (22) may be formed of stainless steel (e.g., a stainless steel hypotube, etc.) and/or any other suitable rigid material.

As shown best in FIG. 2, cannula shaft (22) includes a lumen (30) that extends proximally from open distal end (26) and opens to a hollow interior (32) of grip portion (24). Lumen (30) may be formed with any suitable diameter. By way of example only, lumen (30) may be formed with a diameter of approximately 2.44 mm. Additionally, cannula shaft (22) and lumen (30) may be formed with a transverse cross-sectional profile of any suitable shape, such as a circular or a non-circular shape. It will be appreciated that a non-circular profile, such as an elliptical profile, may allow additional clearance within an anatomical passageway, such as a nasal passageway, for positioning other instruments within the passageway alongside cannula shaft (22).

Grip portion (24) of surgical instrument (12) of the present example has a generally rectangular, tab-like body configured to be gripped by an operator between two or more digits, such as a thumb and a finger, for example. Cannula shaft (22) extends distally from a distal end of grip portion (24), and a suction conduit port (34) is coupled to a proximal end of grip portion (24). Suction conduit port (34) is configured to couple with suction conduit (90), to thereby fluidly couple suction source (14) with surgical instrument (12). In the present example, port (34) has a barbed configuration to promote a secure fit with suction conduit (16), which may be elastomeric. It should be understood that various other types of port configurations may be employed in other examples. As seen in FIG. 2, cannula lumen (30), grip portion interior (32), suction conduit port (34), and suction conduit (16) are in fluid communication with one another and cooperate to provide an unobstructed fluid flow path from open distal end (26) of cannula shaft (22) to suction conduit (16) and ultimately suction source (14).

As shown best in FIG. 2, tab-like body of grip portion (24) includes an upper surface (36) configured to be engaged by a first digit of an operator, such as a thumb, and an opposed lower surface (38) configured to be engaged by a second digit of an operator, such as a finger. A transverse vent opening (40) is formed in upper surface (36) and opens to hollow interior (32) of grip portion (24), such that vent opening (40) is in fluid communication with the fluid flow path of surgical instrument system (10).

In use, an operator may selectively vary the amount of suction applied at open distal end (26) of cannula shaft (22) by adjusting the amount of vent opening (40) that is exposed to the ambient environment. For example, the operator may increase the amount of suction applied at open distal end (26) by closing more or all of vent opening (40) with a thumb or finger. Conversely, the operator may decrease the amount of suction applied at open distal end (26) by partially removing the thumb or finger from vent opening (40), thereby causing suction source (14) to draw air from the ambient environment through vent opening (40). Vent opening (40) may be configured such that fully exposing vent opening (40) to the ambient environment, by fully removing the thumb or finger from vent opening (40), causes suction at open distal end (26) to drop to zero. In the present example, vent opening (40) is formed with a teardrop shape, though vent opening (40) may be formed with various other suitable shapes in other examples. By way of example only, the teardrop shape (or some other elongate shape) may enable the operator to selectively vary the amount of suction at open distal end (26) of cannula shaft (22) based on the longitudinal position of the operator's thumb (or other finger) on vent opening (40).

Upper and lower surfaces (36, 38) of grip portion (24) may be configured to promote gripping of grip portion (24) by an operator. In particular, in the present example upper surface (36) provides a concave contour while lower surface (38) provides a series of ridges. By way of example only, an operator may grasp grip portion (24) by placing a thumb on upper surface (36) and the side of the index finger of the same hand on lower surface (38). The rectangular shape of grip portion (24) may provide the operator with substantial purchase on grip portion (24), while the configurations of surfaces (36, 38) may further secure the operator's grip. Surgical instrument (12) and its gripping and suction features may be further configured in accordance with one or more teachings of U.S. Pat. App. No. 62/512,830, entitled "Navigable Suction Instrument With Coaxial Annular Sensor," filed May 31, 2017, the disclosure of which is incorporated by reference herein.

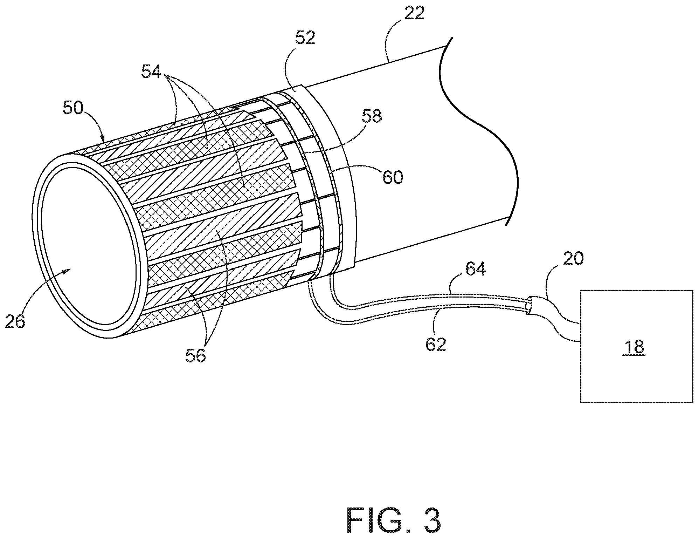

As shown in FIGS. 1 and 3, surgical instrument (12) further includes an RF cuff (50) that encircles cannula shaft (22) at open distal end (26). In the present example, cuff (50) includes a cylindrical body (52) and an array of RF electrodes arranged circumferentially about body (52) and which are configured to deliver bipolar RF energy to tissue contacted by cuff (50). In particular, cuff (50) includes a first plurality of positive electrodes (54) and a second plurality of negative electrodes (56) arranged in a circumferentially alternating manner such that each positive electrode (54) is positioned circumferentially adjacent to at least one negative electrode (56). In the present example, cuff body (52) and electrodes (54, 56) extend about a full circumference of cannula shaft (22). In other examples, electrodes (54, 56) and/or cuff body (52) may extend only partially about the circumference of cannula shaft (22). Additionally, cuff (50) of the present example is shown having at least four electrodes (54, 56) in each circumferential quadrant, such that cuff (50) includes at least sixteen electrodes (54, 56) about its full circumference. It will be appreciated that in other examples cuff (50) may include any suitable quantity of electrodes (54, 56), such as more than 16 electrodes or fewer than 16 electrodes. For instance, cuff (50) may include at least one positive electrode (54) and at least one negative electrode (56).

Positive electrodes (54) of RF cuff (50) are electrically isolated from negative electrodes (56), such that an electrical coupling between any one positive electrode (54) with any one negative electrode (56) is established only when the positive and negative electrodes (54, 56) simultaneously electrically couple with the same conductive medium, such as tissue, as described below. In the present example, positive electrodes (54) are electrically coupled to a first annular conductive member (58) of cuff (50), and negative electrodes (56) are electrically coupled to a second annular conductive member (60) of cuff (50). Annular conductive members (58, 60) are arranged at a proximal end of cuff body (52) and are electrically isolated from one another. First annular conductive member (58) is electrically coupled to a first electrical lead (62), and second annular conductive member (60) is electrically coupled to a second electrical lead (64).

In the present example, electrical leads (62, 64) are arranged externally of cannula shaft (22) and are housed within wire (20) that electrically couples electrodes (54, 56) with generator (18). In some versions, electrical leads (62, 64) may be arranged within and extend proximally through cannula lumen (30), or be integrated within a sidewall of cannula shaft (22), for example. In such configurations, surgical instrument (12) may include an electrical connector (not shown) arranged on a proximal portion of instrument (12), such as on or near grip portion (24) for example. Such an electrical connector may function to electrically couple surgical instrument (12) with generator (18) via an external cable or wire, similar to wire (20) for example. In other examples, generator (18) may be in the form of a battery (not shown) housed within a body of surgical instrument (12) such that no external wires or electrical connectors are required. Other suitable ways in which generator (18) may be configured, and other suitable ways in which generator (18) may be coupled with electrodes (54, 56), will be apparent to those of ordinary skill in the art in view of the teachings herein. It should also be understood that a switch (e.g., button on grip portion (24), footswitch, button on generator (18)) may be provided to enable the operator to selectively activate and deactivate electrodes (54, 56) as needed.

Electrodes (54, 56) of the present example are shown in the form of elongate rectangular members arranged in a single ring extending about a circumference of cuff body (52) and cannula distal end (26). In other examples, electrodes (54, 56), may be provided with various other suitable shapes and arranged in various other suitable configurations. For instance, though not shown, cuff (50) may include multiple rings of electrodes (54, 56), each ring having a circumferentially alternating arrangement of positive and negative electrodes (54, 56). Alternatively, or in addition, cuff (50) may include one or more rings of one or more positive electrodes (54), and one or more adjacent rings of one or more negative electrodes (56), such that each ring has a single polarity, with the different rings being longitudinally spaced apart from each other. In such a configuration, electrode polarities would alternate in an axial direction rather than, or in addition to, a circumferential direction.

As shown in FIG. 3, cuff (50) of the present example is arranged at the distal end (26) of cannula shaft (22) such that the distal ends of electrodes (54, 56) extend fully to cannula distal end (26). In other examples, cuff (50) may be spaced proximally from cannula distal end (26). Additionally, one or more cuffs (50) may be provided along a length of cannula shaft (22). In some examples, cuff (50) may be selectively removable from cannula shaft (22). In other examples, cuff (50) may be permanently attached to cannula shaft (22). In further examples, cuff (50) may be integrated within the structure of cannula shaft (22), such that electrodes (54) are generally flush with the exterior surface of cannula shaft (22). For instance, cuff body (52) may be omitted and electrodes (54, 56) and conductive members (58, 60) may be provided by cannula shaft (22) itself.

While RF cuff (50) is described herein as a component of suction instrument (12), it should be understood that RF cuff (50) may be incorporated into various other surgical instruments. For instance, and by way of example only, RF cuff (50) with or without its cylindrical body (52) may be provided on catheters, dilation catheters, guide catheters, guide wires, and any other suitable surgical instrument having an elongate body on which a circumferential arrangement of electrodes (54, 56) of cuff (50) may be applied. Accordingly, the teachings provided herein are not limited to suction instruments and operations per se. Other suitable instruments and procedures in which the teachings herein may be applied will be apparent to those of ordinary skill in the art. By way of example only, such instruments and procedures may include those disclosed in U.S. Pat. Pub. No. 2017/0273747, entitled "Apparatus and Method for Treatment of Ethmoid Sinus," published September 28, 2017, the disclosure of which is incorporated herein; U.S. Pat. Pub. No. 2017/0119414, entitled "Fluid Communication Features for Eustachian Tube Dilation Instrument," published May 4, 2017, the disclosure of which is incorporated herein; U.S. Pat. Pub. No. 2017/0056632, entitled "Dilation Catheter with Expandable Stop Element," published March 2, 2017, the disclosure of which is incorporated herein; U.S. Pat. No. 9,155,492, entitled "Sinus Illumination Lightwire Device," issued October 13, 2015, the disclosure of which is incorporated by reference herein; and U.S. Pat. Pub. No. 2011/0004057, entitled "Systems and Methods for Transnasal Dilation of Passageways in the Ear, Nose or Throat," published January 6, 2011, the disclosure of which is incorporated by reference herein.

### B. Exemplary Method of Using Surgical Instrument System

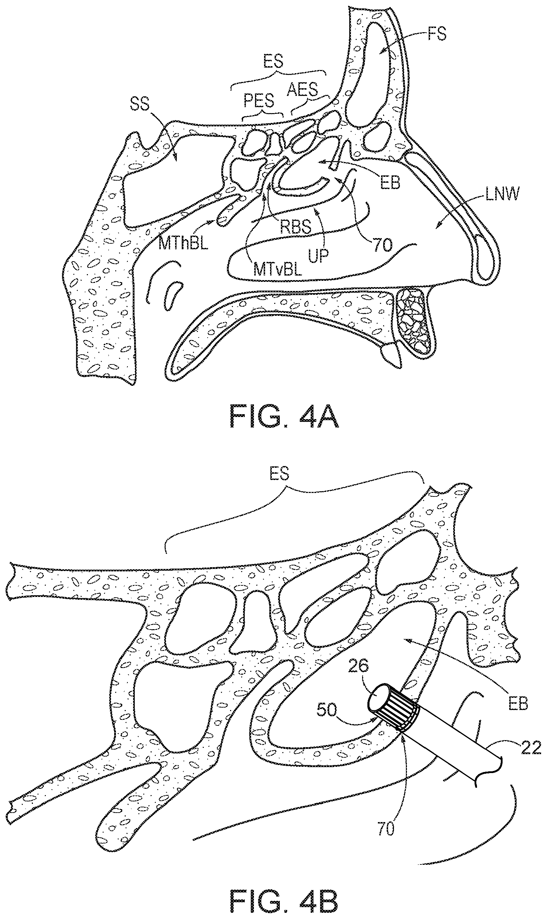

FIGS. 4A and 4B show an exemplary method of using surgical instrument system (10) described above to perform a surgical procedure involving suction of fluids and sealing of tissue. FIG. 4A shows a left sagittal sectional view of a portion of a patient's head, which includes a sphenoid sinus (SS), an ethmoid sinus (ES), a frontal sinus (FS), a middle turbinate horizontal basal lamella (MThBL), a middle turbinate vertical basal lamella (MTvBL), a uncinate process (UP), and a lateral nasal wall (LNW). The ethmoid sinus (ES) comprises a set of sinus cells that may be categorized as anterior ethmoid sinus (AES) cells and posterior ethmoid sinus (PES) cells. The ethmoid bulla (EB) is the largest ethmoid sinus (ES) cell, and is generally inferior and anterior to the other cells of the ethmoid sinus (ES). The posterior wall of the ethmoid bulla (EB) and the middle turbinate vertical basal lamella (MTvBL) together define a retrobullar space (RBS). It should be understood that anatomical variation among patients is such that this retrobullar space (RBS) may or may not be present in a given individual.

The ethmoid sinus (ES) includes ostia (not shown) for providing fluid communication to and from the cells of the ethmoid sinus (ES) and the nasal cavity. For instance, ostia may provide fluid paths for cells within the anterior ethmoid sinus (AES), cells within the posterior ethmoid sinus (PES), and the ethmoid bulla (EB). In some instances, suprabullar cells of the ethmoid sinus (ES) drain into the ethmoid bulla (EB). Some suprabullar cells may drain directly into the retrobullar space (RBS). The ethmoid bulla (EB) may itself provide fluid communication with the nasal cavity via one or more ostia, such that the ethmoid bulla (EB) may provide a fluid communication path between the other ethmoid sinus (ES) cells (that drain into the ethmoid bulla (EB)) and the nasal cavity. For instance, the ethmoid bulla (EB) may provide fluid communication through an ostium at the retrobullar space (RBS). The fluid communication paths provided by ostia may allow the entry of air and liquids (e.g., medications); while also allowing drainage of mucus. In some instances, the ostia may become blocked, may become functionally closed due to mucosal thickening, or may otherwise not provide sufficient fluid communication. In addition, or in the alternative, the configuration of the retrobullar space (RBS) may impede flow through the ostium of the ethmoid bulla (EB).

The anatomy of the ethmoid sinus (ES) may make it impractical to perform a dilation procedure on ostia of the ethmoid sinus (ES) using a dilation catheter to improve fluid communication within the ethmoid sinus (ES). This may lead some physicians to perform an ethmoidectomy, which is an invasive procedure that involves removal of ethmoid sinus (ES) portions (e.g., tissue and bone) using an instrument such as a debriding instrument. This kind of procedure may be somewhat crude an inelegant, resulting in removal of significant amounts of mucosa that might otherwise benefit the patient. Ethmoidectomy procedures may also have risks of inadvertent damage to optic nerves, damage to orbital muscles, damage to olfactory bulbs, damage to other anatomical structures, and even leakage of cerebrospinal fluid. Even in successful ethmoidectomies, the patient may need to return for several follow-up debridements. It may therefore be desirable to improve fluid communication from within the ethmoid sinus (ES) to the nasal cavity without resorting to a procedure like an ethmoidectomy. In some instances, this may involve the formation of an opening in one or more cells of the ethmoid sinus (ES) using a surgical instrument.

In the present example shown in FIGS. 4A and 4B, an opening (70) is formed in the anterior wall of the ethmoid bulla (EB) to facilitate drainage of the ethmoid bulla (EB). Opening (70) may be formed using any suitable surgical instrument and process, such as an instrument and process of the types disclosed in U.S. Pat. Pub. No. 2017/0273747, incorporated by reference above. Formation of opening (70) may cause the surrounding tissue to bleed into the ethmoid bulla (EB) and adjacent portions of ethmoid sinus (ES), which maybe undesirable. As described below, surgical instrument (12) maybe employed to drain fluids from the ethmoid bulla (EB) and simultaneously seal the tissue surrounding opening (70) to mitigate further bleeding.

As shown in FIG. 4B, distal end (26) of cannula shaft (22) of surgical instrument (12) is inserted into the nasal cavity of the patient and through opening (70) formed in the wall of the ethmoid bulla (EB). Once suitably positioned as shown in FIG. 4B, the operator may at least partially close vent opening (40) of grip portion (24) of instrument (12) to activate suction at open distal end (26) and thereby draw fluids out of the ethmoid bulla (EB). Simultaneously, or before or after this suction step, the operator may activate instrument (12) to deliver bipolar RF energy to RF cuff (50). Specifically, surgical instrument (12) may be manipulated by the operator such that the bleeding tissue is positioned in contact with at least one positive electrode (54) and at least one negative electrode (56) simultaneously so that RF energy delivered to cuff (50) from generator (18) passes through the tissue and thereby seals the tissue. The operator may move cannula shaft (22) in rotational and/or axial directions repeatedly as needed to ensure that a full perimeter of opening (70) is contacted and sealed by energized electrodes (54, 56) of cuff (50).

While surgical instrument system (10) is shown in FIGS. 4A and 4B as being implemented in connection with an ethmoid sinus procedure, it will be appreciated that surgical instrument system (10) may be employed in various other surgical procedures for treating a nasal passage and/or other portions of the patient body. Moreover, as described above, it will be appreciated that RF cuff (50) may be provided on various types of elongate surgical instruments other than suction instruments of the type described herein.

### II. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A surgical instrument, comprising: (a) an elongate body configured to be inserted through the nasal cavity of a patient; and (b) a plurality of electrodes spaced circumferentially about an exterior of the elongate body, wherein the electrodes are configured to deliver bipolar RF energy to tissue for sealing the tissue.

### Example 2

The surgical instrument of Example 1, wherein the elongate body is rigid.

### Example 3

The surgical instrument of any of the preceding Examples, wherein the elongate body comprises a shaft defining a lumen.

### Example 4

The surgical instrument of Example 3, wherein the shaft includes a straight portion and a bent portion.

### Example 5

The surgical instrument of any one or more of Examples 3 through 4, wherein the surgical instrument further comprises a grip portion, wherein the shaft extends distally from the grip portion.

### Example 6

The surgical instrument of any one or more of Examples 3 through 5, wherein a proximal end of the surgical instrument is configured to operatively couple with a suction source, wherein the surgical instrument is operable to draw fluid proximally through the lumen with suction provided by the suction source.

### Example 7

The surgical instrument of any of the preceding Examples, wherein the surgical instrument is operable to draw fluid proximally through a distal end of the surgical instrument with suction and simultaneously deliver bipolar RF energy via the electrodes to tissue for sealing the tissue.

### Example 8

The surgical instrument of any of the preceding Examples, wherein the electrodes are arranged at a distal end of the elongate body.

### Example 9

The surgical instrument of any of the preceding Examples, wherein the electrodes are arranged circumferentially with alternating polarities.

### Example 10

The surgical instrument of any of the preceding Examples, wherein the electrodes include a first set of electrodes having a first polarity and a second set of electrodes having a second polarity, wherein the electrodes of the first set are electrically isolated from the electrodes of the second set.

### Example 11

The surgical instrument of any of Example 10, wherein the electrodes of the first set are electrically coupled with a first electrical lead, wherein the electrodes of the second set are electrically coupled with a second electrical lead, wherein the first and second electrical leads are configured to electrically couple with a power source.

### Example 12

The surgical instrument of any of the preceding Examples, wherein the electrodes are provided by a cuff that encircles the elongate body.

### Example 13

The surgical instrument of Example 12, wherein the cuff is arranged at a distal end of the elongate body.

### Example 14

The surgical instrument of any of the preceding Examples, wherein the plurality of electrodes includes at least four electrodes.

### Example 15

A surgical system, comprising: (a) the surgical instrument of claim 1, wherein the surgical instrument includes a lumen; (b) a suction source operatively coupled with the surgical instrument, wherein the suction source is operable to apply suction to the surgical instrument for drawing fluid proximally through the lumen; and (c) a power source operatively coupled with the surgical instrument, wherein the power source is operable to energize the electrodes with bipolar RF energy for sealing tissue.

### Example 16

A surgical instrument, comprising: (a) an elongate body configured to be inserted through the nasal cavity of a patient; and (b) first and second electrodes arranged at a distal end of the elongate body, wherein the first and second electrodes are configured to deliver bipolar RF energy to tissue for sealing the tissue.

### Example 17

The surgical instrument of Example 16, wherein the elongate body comprises a shaft having a lumen and an open distal end, wherein the first and second electrodes are spaced circumferentially about the open distal end.

### Example 18

The surgical instrument of Example 17, wherein the surgical instrument is operable to draw fluid proximally through the lumen with suction.

### Example 19

A surgical instrument, comprising: (a) a rigid shaft configured to be inserted through the nasal cavity of a patient, wherein the rigid shaft has an open distal end and a lumen, wherein the surgical instrument is operable to draw fluid proximally through the open distal end and the lumen with suction; and (b) at least one electrode provided on an exterior of the rigid shaft, wherein the at least one electrode is configured to deliver RF energy to tissue for sealing the tissue.

### Example 20

The surgical instrument of Example 19, wherein the at least one electrode comprises a plurality of electrodes, wherein the electrodes are configured to deliver bipolar RF energy to tissue for sealing the tissue.

### III. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument, comprising:
(a) an elongate body configured to be inserted through the nasal cavity of a patient; and
(b) a plurality of electrodes spaced circumferentially about an exterior of the elongate body, wherein the electrodes are configured to deliver bipolar RF energy to tissue for sealing the tissue.

2. The surgical instrument of claim 1, wherein the elongate body is rigid.

3. The surgical instrument of claim 1, wherein the elongate body comprises a shaft defining a lumen.

4. The surgical instrument of claim 3, wherein (i) the shaft includes a straight portion and a bent portion; (ii) the surgical instrument further comprises a grip portion, wherein the shaft extends distally from the grip portion; or (iii) a proximal end of the surgical instrument is configured to operatively couple with a suction source, wherein the surgical instrument is operable to draw fluid proximally through the lumen with suction provided by the suction source.

5. The surgical instrument of claim 1, wherein the surgical instrument is operable to draw fluid proximally through a distal end of the surgical instrument with suction and simultaneously deliver bipolar RF energy via the electrodes to tissue for sealing the tissue.

6. The surgical instrument of claim 1, wherein the electrodes are arranged at a distal end of the elongate body, or wherein the electrodes are arranged circumferentially with alternating polarities.

7. The surgical instrument of claim 1, wherein the electrodes include a first set of electrodes having a first polarity and a second set of electrodes having a second polarity, wherein the electrodes of the first set are electrically isolated from the electrodes of the second set.

8. The surgical instrument of claim 7, wherein the electrodes of the first set are electrically coupled with a first electrical lead, wherein the electrodes of the second set are electrically coupled with a second electrical lead, wherein the first and second electrical leads are configured to electrically couple with a power source.

9. The surgical instrument of claim 1, wherein the electrodes are provided by a cuff that encircles the elongate body, preferably wherein the cuff is arranged at a distal end of the elongate body.

10. The surgical instrument of claim 1, wherein the plurality of electrodes includes at least four electrodes.

11. A surgical system, comprising:
(a) the surgical instrument of claim 1, wherein the surgical instrument includes a lumen;
(b) a suction source operatively coupled with the surgical instrument, wherein the suction source is operable to apply suction to the surgical instrument for drawing fluid proximally through the lumen; and
(c) a power source operatively coupled with the surgical instrument, wherein the power source is operable to energize the electrodes with bipolar RF energy for sealing tissue.

12. A surgical instrument, comprising:
(a) an elongate body configured to be inserted through the nasal cavity of a patient; and
(b) first and second electrodes arranged at a distal end of the elongate body, wherein the first and second electrodes are configured to deliver bipolar RF energy to tissue for sealing the tissue.

13. The surgical instrument of claim 12, wherein the elongate body comprises a shaft having a lumen and an open distal end, wherein the first and second electrodes are spaced circumferentially about the open distal end, preferably wherein the surgical instrument is operable to draw fluid proximally through the lumen with suction.

14. A surgical instrument, comprising:
(a) a rigid shaft configured to be inserted through the nasal cavity of a patient, wherein the rigid shaft has an open distal end and a lumen, wherein the surgical instrument is operable to draw fluid proximally through the open distal end and the lumen with suction; and
(b) at least one electrode provided on an exterior of the rigid shaft, wherein the at least one electrode is configured to deliver RF energy to tissue for sealing the tissue.

15. The surgical instrument of claim 14, wherein the at least one electrode comprises a plurality of electrodes, wherein the electrodes are configured to deliver bipolar RF energy to tissue for sealing the tissue.
